(19) Europäisches Patentamt
European Patent Office
Office européen des brevets

(11) EP 0 339 618 B1

(12) EUROPEAN PATENT SPECIFICATION

(45) Date of publication and mention of the grant of the patent: **19.08.1998 Bulletin 1998/34**

(51) Int Cl.[6]: **C07C 255/12**, C07C 235/06, C07C 59/10, C07C 253/14

(21) Application number: **89107575.6**

(22) Date of filing: **26.04.1989**

# (54) Method for preparing optically active 3,4-dihydroxy butyric acid derivatives

Verfahren zur Herstellung von optisch aktiven 3,4-Dihydroxybuttersäurederivaten

Procédé de préparation de dérivés de l'acide 3,4-dihydroxybutyrique optiquement actifs

(84) Designated Contracting States:
**BE CH DE ES FR GB IT LI**

(30) Priority: **27.04.1988 JP 106856/88**
**31.03.1989 JP 82466/89**

(43) Date of publication of application:
**02.11.1989 Bulletin 1989/44**

(73) Proprietor: **KANEGAFUCHI KAGAKU KOGYO KABUSHIKI KAISHA**
**Kita-ku, Osaka-shi, Osaka 530 (JP)**

(72) Inventors:
- **Inoue, Kenji**
  **Kako-gun Hyogo-ken (JP)**
- **Matsumoto, Mitsunori**
  **Nakatsu-shi Oita-ken (JP)**
- **Takahashi, Satomi**
  **Kobe-shi Hyogo-ken (JP)**

(74) Representative: **Türk, Gille, Hrabal, Leifert**
**Brucknerstrasse 20**
**40593 Düsseldorf (DE)**

(56) References cited:
**EP-A- 0 257 619**

- **BULLETIN DE LA SOCIETE CHIMIQUE DE FRANCE, 1956, pages 1419-1424; R. RAMBAUD et al.: "No. 223. - Contribution à l'étude du buténolide (3e mémoire)"**
- **CHEMICAL ABSTRACTS, vol. 89, no. 13, 25th September 1978, page 845, abstract no. 108744x, Columbus, Ohio, US; & JP-A-78 31 642 (SUMITOMO CHEMICAL CO., LTD) 25-03-1978**
- **CHEMICAL ABSTRACTS, vol. 49, no. 6, 25th March 1955, column 3819, abstract no. c, Columbus, Ohio, US; R. RAMBAUD et al.: "Hydroxybutric derivatives", && COMPT. REND. 238, 1231-2 (1954)**
- **JOURNAL OF THE AMERICAN CHEMICAL SOCIETY, vol. 107, no. 24, 27th November 1985, pages 7008-7018, American Chemical Society; D.C. CRANS et al.: "Glycerol kinase: substrate specificity"**
- **JOURNAL OF THE AMERICAN CHEMICAL SOCIETY, vol. 53, 1931, pages 3164-3171**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

BACKGROUND OF THE INVENTION

Field of the Invention:

The present invention relates to a method for preparing optically active 3,4-dihydroxy butyronitrile and 3,4-dihydroxy butyric acid and 3,4-dihydroxy butyric acid amide.

Description of the Prior Art:

Hitherto the following two methods have been known as the preparation of optically active 3,4-dihydroxy butyric acid derivatives or 3,4-dihydroxy butyronitrile:

(1) The method of reducing dimethyl ester of L-malic acid by using boran dimethyl sulfide in the presence of a catalytic amount of sodium borohydride to convert into 3,4-dihydroxy butyric acid methyl ester (Japanese Laid-Open Patent 61-22049, Japanese Laid-Open Patent 63-22056, Chemistry Letters 1389, (1984); and
(2) The method of selectively hydrolyzing dimethyl ester of L-malic acid by using esterase (PLE), reducing by using boran dimethyl sulfide, and converting into 3,4-dihydroxy butyric acid methyl ester (Journal of Organic Chemistry, 50, 1145, (1985)).

On the other hand, as the preparation of racemic compounds, the following methods, among others, are known:

(1) The method of producing 3,4-dihydroxy butyronitrile by the reaction of racemic 3-chloro-1,2-propanediol with KCNin water, or successively treating with NaOH and HCl to convert into (S)-butyrolactone derivative (Compt. Rend. 238, 1231, (1954)).
(2) The method of causing racemic 3-chloro-1,2-propanediol to react with NaCN in water, and refining by using an ion exchange resin (J. Am. Chem. Soc., 107, 24, p. 7018 (1985)).

On these methods, the methods of producing optically active compounds use L-malic acid as the starting material, and also require boran dimethyl sulfide which is a relatively expensive reducing agent, and hence they involve various problems in economy and operation as the practical preparation of optically active 3,4-dihydroxy butyric acid derivatives.

On the other hand, the method of production of racemic 3,4-dihydroxy butyronitrile by cyanation of 3-chloro-1,2-propanediol is likely to produce by-products such as 3,4-dihydroxy amide and 3,4-dihydroxy butyric acid, and is poor in selectivity of reaction, and it involves various problems to be solved in the reaction selectivity, reaction yield and controllability, as the practical preparation of 3, 4-dihydroxy butyronitrile.

Chemical Abstracts Vol. 89, No. 108744x describes the use of lower trialkylamines as catalyst in the preparation of unsubstituted or ring-halo-substituted benzyl cyanides by the reaction of benzyl halides with alkali cyanides.

Journal of the American Chemical Society Vol. 53 (24), 1931, p.3164 - 3171 reports about the preparation of hydroxy-3-butanolide by means of double decomposition between CNK and chloro-1-propanediol.

SUMMARY OF THE INVENTION

It is hence a primary object of the invention to present a method for preparing optically active 3,4-dihydroxy butyronitrile and 3,4-dihydroxy butyric acid derivatives, both economically and efficiently.

Other objects and benefits of the invention will be better understood and appreciated from the following detailed description.

The present inventors intensively researched in order to establish an industrial preparation method of optically active (S)-3,4-dihydroxy butyronitrile and (S)-3,4-dihydroxy butyric acid derivatives, both economically and efficiently so as to achieve the above objects, and discovered a method of producing (S)-3,4-dihydroxy butyronitrile and its hydration reaction product or hydrolysis reaction product, (S)-3,4-dihydroxy butyric amide, (S)-3,4-dihydroxy butyric acid, selectively by one pot, by controlling the reaction conditions, by making use of the reaction of cyanating agent such as NaCN and KCN with (R)-3-chloro-1,2-propanediol which can be efficiently manufactured by stereo-selective microorganism decomposition of racemic 3-chloro-1,2-propanediol (Japanese Laid-Open Patents 62-122597, 62-158494, 63-36978), thereby completing this invention.

## DETAILED DESCRIPTION OF THE INVENTION

A first invention relates to a method for reacting (R)-3-chloro-1,2-propanediol represented by the structural formula (I)

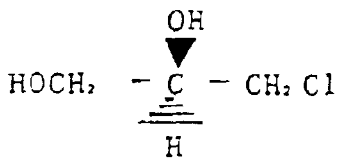
( I )

with a cyanating agent, wherein, by controlling a reaction temperature, an amount of a base as required and a ratio of water in alcohol and/or water solvent, any one of (S)-3,4-dihydroxy butyronitrile represented by the structural formula (II)

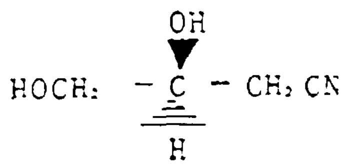
( II )

(S)-3,4-dihydroxy butyric acid amide represented by structural formula (III)

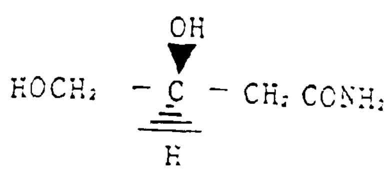
( III )

and (S)-3,4-dihydroxy butyric acid represented by structural formula (IV)

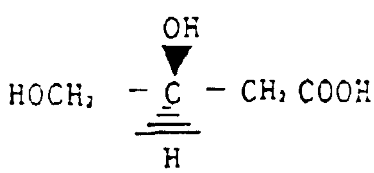
( IV )

is selectively produced.

A further embodiment relates to a method for preparing selectively (S)-3,4-dihydroxy butyronitrile represented by the structural formula (II)

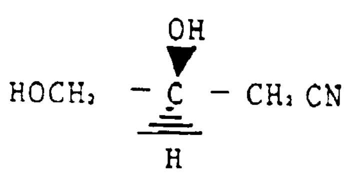
( II )

by reacting (R)-3-chloro-1,2-propanediol represented by the strutural formula (I)

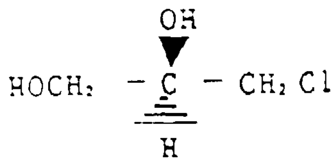
(I)

with a cyanating agent, wherein the reaction is conducted at a reaction temperature of 20 - 40 °C, in the presence of 0,1 - 2 molar equivalent base and in methanol as a solvent.

A preferred embodiment relates to a method for preparing selectively (S)-3,4-dihydroxy butyric acid amide represented by structural formula (III)

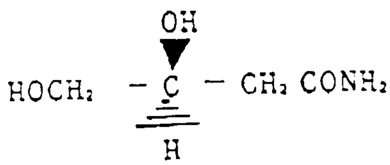
(III)

by reacting (R)-3-chloro-1,2-propanediol represented by the structural formula (I)

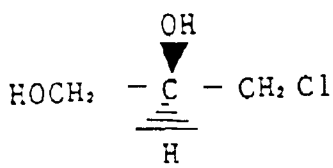
(I)

with a cyanating agent, wherein the reaction is conducted at a reaction temperature of 30 - 60 °C, in water as a solvent and without addition of a base.

A further embodiment relates to a method for preparing selectively (S)-3,4-dihydroxy butyric acid represented by structural formula (IV)

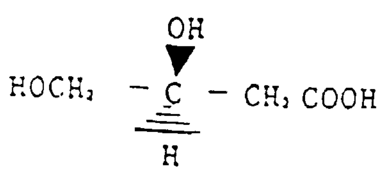
(IV)

by reacting (R)-3-chloro-1,2-propanediol represented by the structural formula (I)

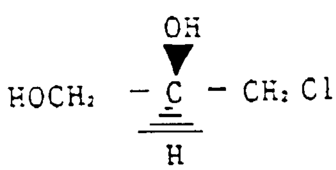
(I)

with a cyanating agent, wherein the reaction is conducted at a temperature of 40 - 80 °C, in the presence of 1 - 3 molar equivalent base and in water as a solvent.

In the reaction to obtain (S)-3,4-dihydroxy butyronitrile (II) from (R)-3-chloro-1,2-propanediol (I), alcohol such as methanol and ethanol, and water can be used as a solvent either alone or as a mixture, but in order to minimize the

formation of by-products such as 3,4-dihydroxy butyric amide and to enhance the selectivity for formation of 3,4-dihydroxy butyronitrile, it is preferable to perform the reaction in methanol or in a mixed solvent of alcohol and water. As cyanating agents, NaCN, KCN, HCN, $MgCN_2$, AgCN, CuCN, etc. may be used, and in particular, NaCN, KCN and other alkali metal cyanides are preferable from the viewpoint of yield and economy.

The cyanating reaction proceeds only in the presence of an (R)-3-chloro-1,2-propanediol solvent and a cyanating agent, but elimination rate of the starting material may be raised and the selectivity of formation of 3,4-dihydroxy butyronitrile may be enhanced by adding a base to the reaction system, for example, NaOH, KOH or other alkali metal hydroxides, amines, etc., or specifically by adding tertiary amines such as triethylamine by 0.1 to 2 equivalents to the reaction system, preferably 0.5 to 1.5 equivalents. Furthermore, by performing reaction by using triethylamine in methanol, more favorable results will be otained. A proper temperature range for this reaction is 20 to 100°C, but if the temperature is higher, although the elimination of the starting material (R)-3-chloro-1,2-propanediol is faster, the selectivity of reaction tends to be lower, and in order to obtain 3,4-dihydroxy butyronitrile selectively, it is preferable to agitate for 1 to 40 hours until the starting material is nearly eliminated at 20 to 40°C. For isolation and refining, a mineral acid is added to neutralize the reaction solution, and the solvent is replaced by water, and the solvent is distilled away by extracting continuously by using ethyl acetate or the like, so that an oily matter containing mainly (S)-3,4-dihydroxy butyronitrile is obtained. To refine further, the object will be achieved by ordinary distillation or column chromatography. However, the reaction and isolation operations are not limited to these methods, but various methods may be employed. The (S)-3,4-dihydroxy butyronitrile obtained in this way may be easily converted into (S)-3,4-dihydroxy butyric acid amide and (S)-3,4-dihydroxy butyric acid by controlling the conditions in the ordinary nitrile hydrolysis conditions.

The reaction for obtaining selectively (S)-3,4-dihydroxy butyric acid amide from (R)-3-chloro-1,2-propanediol is effected by using water or a mixture of water and alcohol, preferably water as the solvent, and causing (R)-3-chloro-1,2-propanediol to react with the cyanating agent such as NaCN, KCN, at 20 to 80°C, preferably 30 to 60°C, while stirring for 1 to 30 hours, preferably 2 to 20 hours. This reaction tends to produce 3,4-dihydroxy butyric acid as by-product when a base is added to the reaction system or when the temperature is higher than necessary. Isolation and refining operation can be conducted by neutralizing by adding, for example, a mineral acid to the reaction solution, removing the solvent, and performing column chromatography. The amide obtained in this manner may be easily converted into (S)-3,4-dihydroxy butyric acid in the ordinary conditions for amide hydrolysis.

In the reaction for selectively obtaining (S)-3,4-dihydroxy butyric acid from (R)-3-chloro-1,2-propanediol, the object may be achieved by using water or a mixture of water and alcohol, preferably water as the solvent, and stirring at 20 to 100°C, preferably 40 to 80°C, for 30 minutes to 50 hours, preferably 1 to 20 hours, in the presence of the cyanating agent such as NaCN and KCN and preferably base such as NaOH and KOH by 1 to 3 equivalents, preferably 1 to 1.5 equivalents. Isolating and refining operation can be done by neutralizing the reaction solution by a mineral acid or an ion exchange resin, distilling off the solvent, and performing column chromatography, but the operation of reaction and isolation is not limited to these methods, various methods being also employed.

According to this invention, optically active 3,4-dihydroxy butyronitrile and 3,4-dihydroxy butyric acid derivatives may be produced economically and efficiently.

This invention is further described below while referring to examples and reference examples, but they are not intended to limit the invention in any respect.

EXAMPLE 1 : Synthesis of (S)-3,4-dihydroxy butyronitrile

33.2 g of (R)-3-chloro-1,2-propanediol and 30.4 g of triethylamine were dissolved in 90 ml of methanol, and 18.6 g of NaCN was added. After stirring for 20 hours at 30°C, concentrated HCl was slowly dropped while cooling the mixture to 0°C in order to neutralize the excess base, and methanol was distilled away. To the obtained solution, 200 ml of water was added, and extraction was conducted continuously by using ethyl acetate, and the solvent was distilled away, and the obtained oily matter was distilled (B.P. 140 to 150°C/3 mmHg), and finally 25.9 g of (S)-3,4-dihydroxy butyronitrile was obtained.

$[\alpha]_D^{20} = 24.1$ (C = 1.02, $CH_3OH$)

$^1$HNMR ($CDCl_3$, $CD_3OD$) : $\delta$ 2.57 - 2.73 (m, 2H), 3.62 (d, 2H, J = 5 Hz), 3.8 - 4.13 (m, 1H), 4.5 (bs, 2H), IR ($Cm^{-1}$) : (neat) 3400, 2925, 2250, 1415, 1100, 1042

EXAMPLE 2 : Synthesis of (S)-3,4-dihydroxy butyronitrile

After dissolving 18.6 g of NaCN into 90 ml of methanol, a solution having 30.4 g of triethylamine and 33.2 g of (R)-3-chloro-1,2-propanediol dissolved in 20 ml of methanol was added. After stirring for 6 hours at 40°C, concentrated $H_2SO_4$ was slowly added while cooling to 0°C to neutralize the excess base, and the methanol was distilled away, and 300 ml of acetone was added to the obtained solution. Filtering the precipitating solid, the filtrate was concentrated under reduced pressure, and the obtained oily matter was distilled (B.P. 140 to 150°C/3 mmHg), and finally 24 g of

(S)-3,4-dihydroxy butyronitrile was obtained.

EXAMPLE 3 : Synthesis of (S)-3,4-dihydroxy butyric acid amide

After dissolving 12.36 g of NaCN into 200 ml of water, 50 ml of aqueous solution of 22.2 g of (R)-3-chloro-1,2-propanediol was slowly dropped, while stirring for 15 hours at 40°C. The solution was neutralized by concentrated HCl while cooling to 0°C, and $C_2H_5OH$ was added to precipitate an inorganic salt, which was filtered, and the filtrate was concentrated, and the obtained oily matter was refined by column chromatography (acetone) of silica gel, and finally 17.3 g of (S)-3,4-dihydroxy butyric acid amide was obtained.

$[\alpha]^{20}_{D}$ = -15.2 (C = 1.09, $CH_3OH$)

$^1$HNMR ($CDCl_3$, $CD_3OD$) : δ 2.37 - 2.53 (m, 2H), 3.56 (d, 2H, J = 5 Hz), 3.88 - 4.23 (m, 1H), 4.60 (bs, 2H), IR ($Cm^{-1}$) : (neat) 3350, 2925, 1670, 1620, 1418, 1095, 1040

EXAMPLE 4 : Synthesis of (S)-3,4-dihydroxy butyric acid

After dissolving 12.36 g of NaCN and 17.17 g of NaOH in 200 ml of water, 50 ml of aqueous solution of 22.2 g of (R)-3-chloro-1,2-propanediol was slowly dropped while stirring for 15 hours at 80°C. After reaction, while cooling the reaction solution to 0°C, 6N HCl was added to adjust the pH to 2.5, and the volatile matter was distilled away, and $C_2H_5OH$ was added, and the precipitating solid matter was filtered. By distilling away $C_2H_5OH$ from the filtrate, a residue was obtained, and it was refined by silica gel column chromatography (hexane : acetone = 1 : 1), and finally 15.8 g of (S)-3,4-dihydroxy butyric acid was obtained.

$[\alpha]^{20}_{D}$ = -27.9 (C = 0.96, $CH_30H$)

$^1$HNMR ($CDCl_3$, $CD_3OD$) : : δ 2.47 - 2.63 (m, 2H), 3.6 (d, 2H, J = 5 Hz), 3.97 - 4.3 (m, 1H), 4.77 - 5.32 (m, 3H) IR ($Cm^{-1}$) : (neat) 3300, 2900, 1710, 1390, 1180, 1030

REFERENCE EXAMPLE 1 : Synthesis of (S)-3,4-dihydroxy butyric acid amide

3 g of (S)-3,4-dihyroxy butyronitrile was dissolved in 30 ml of 1N NaOH, and the solution was stirred for 5 hours at 40°C, and was then cooled to 0°C while neutralizing with 2N HCl. After distilling away the volatile matter, $C_2H_5OH$ was added, and the precipitating solid matter was filtered away, and the oily matter obtained by distilling away $C_2H_5OH$ from the filtrate was refined by silica gel column chromatography (acetone), and finally 3.01 g of (S)-3,4-dihydroxy butyric acid amide was obtained.

REFERENCE EXAMPLE 2 : Synthesis of (S)-3,4-dihydroxy butyric acid

Dissolving 3 g of (S)-3,4-dihydroxy butyronitrile in 30 ml of 2N NaOH, the solution was stirred for 6 hours at 80°C, and it was then cooled to 0°C while adjusting the pH to 2.5 by adding 2N HCl, and by the same process as in EXAMPLE 4 thereafter, 2.92 g of (S)-3,4-dihydroxy butyric acid was obtained.

REFERENCE EXAMPLE 3 : Synthesis of (S)-3,4-dihydroxy butyric acid

Dissolving 3.57 g of (S)-3,4-dihydroxy butyric acid amide in 40 ml of 1N NaOH, the solution was stirred for 5 hours at 80°C, and the solution was cooled to 0°C while adjusting the pH to 2.5 by adding 2N HCl, and by the same process as in EXAMPLE 3 thereafter, 3.28 g of (S)-3,4-dihydroxy butyric acid was obtained.

**Claims**

1. A method for reacting (R)-3-chloro-1,2-propanediol represented by the structural formula (I)

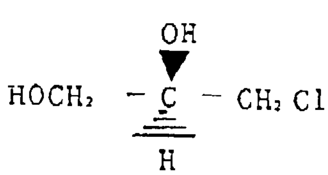

( I )

with a cyanating agent, wherein, by controlling a reaction temperature, an amount of a base as required and a ratio of water in alcohol and/or water solvent, any one of (S)-3,4-dihydroxy butyronitrile represented by the structural formula (II)

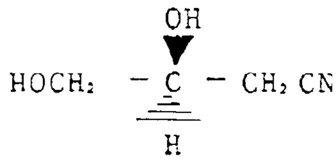

(II)

(S)-3,4-dihydroxy butyric acid amide represented by structural formula (III)

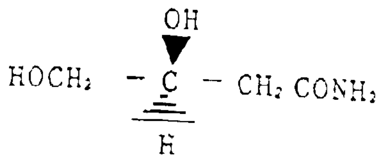

(III)

and (S)-3,4-dihydroxy butyric acid represented by structural formula (IV)

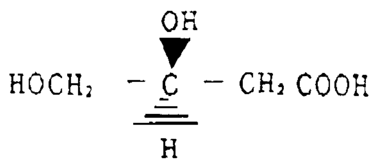

(IV)

is selectively produced.

**2.** A method for preparing selectively (S)-3,4-dihydroxy butyronitrile represented by the structural formula (II)

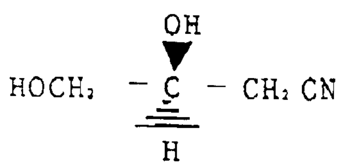

(II)

by reacting (R)-3-chloro-1,2-propanediol represented by the strutural formula (I)

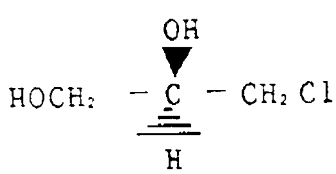

(I)

with a cyanating agent, wherein the reaction is conducted at a reaction temperature of 20 - 40 °C, in the presence of 0,1 - 2 molar equivalent base and in methanol as a solvent.

**3.** A method for preparing selectively (S)-3,4-dihydroxy butyric acid amide represented by strutural fomula (III)

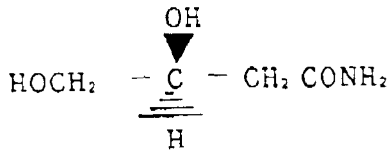

(III)

by reacting (R)-3-chloro-1,2-propanediol represented by the structural formula (I)

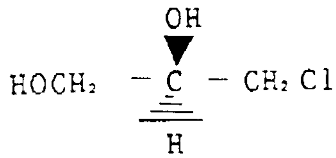

(I)

with a cyanating agent, wherein the reaction is conducted at a reaction temperature of 30 - 60 °C, in water as a solvent and without addition of a base.

4. A method for preparing selectively (S)-3,4-dihydroxy butyric acid represented by structural formula (IV)

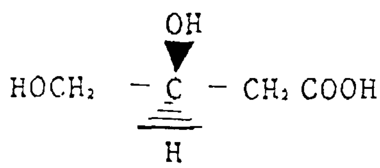

(IV)

by reacting (R)-3-chloro-1,2-propanediol represented by the structural formula (I)

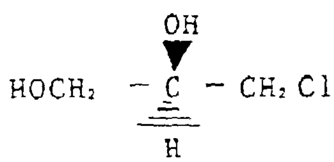

(I)

with a cyanating agent, wherein the reaction is conducted at a temperature of 40 - 80 °C, in the presence of 1 - 3 molar equivalent base and in water as a solvent.

**Patentansprüche**

1. Verfahren zur Umsetzung von durch die Strukturformel (I) dargestelltem (R)-3-Chloro-1,2-propandiol

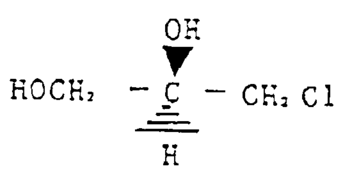

(I)

mit einem Cyanierungsmittel, wobei durch Kontrolle der Reaktionstemperatur, der erforderlichen Menge einer Base und des Verhältnisses von Wasser in Alkohol und/oder eines Wasserlösungsmittels eine der Verbindungen (S)-3,4-Dihydroxybutyronitril, dargestellt durch die Strukturformel (II)

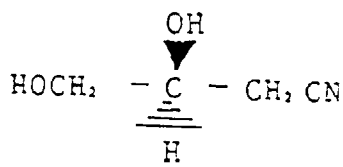
(II)

(S)-3,4-Dihydroxybuttersäureamid, dargestellt durch die Strukturformel (III)

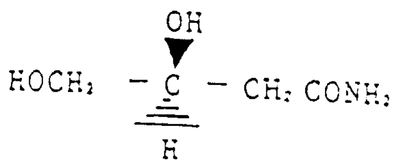
(III)

und (S)-3,4-Dihydroxybuttersäure, dargestellt durch die Strukturformel (IV)

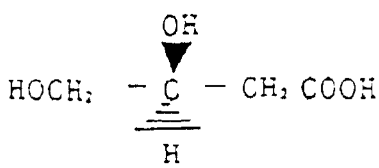
(IV)

selektiv hergestellt wird.

**2.** Verfahren zur selektiven Herstellung von (S)-3,4-Dihydroxybutyronitril, dargestellt durch die Strukturformel (II)

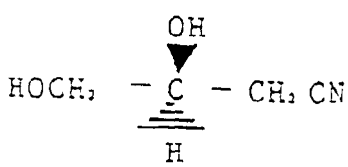
(II)

durch Umsetzen von (R)-3-Chloro-1,2-propandiol, dargestellt durch die Strukturformel (I)

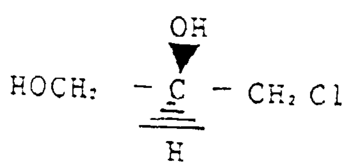
(I)

mit einem Cyanierungsmittel, wobei die Reaktion bei einer Reaktionstemperatur von 20 bis 40°C in Gegenwart

von 0,1 bis 2 molaren Äquivalenten Base und in Methanol als Lösungsmittel durchgeführt wird.

3. Verfahren zur selektiven Herstellung von (S)-3,4-Dihydroxybuttersäureamid, dargestellt durch die Strukturformel (III)

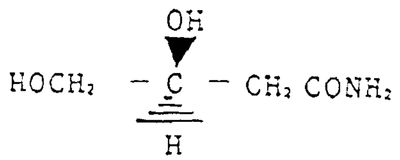

(III)

durch Umsetzen von (R)-3,4-Chloro-1,2-propandiol, dargestellt durch die Strukturformel (I)

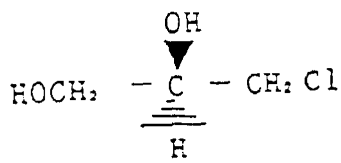

(I)

mit einem Cyanierungsmittel, wobei die Reaktion bei einer Reaktionstemperatur von 30 bis 60°C in Wasser als Lösungsmittel und ohne Zugabe einer Base durchgeführt wird.

4. Verfahren zur selektiven Herstellung von (S)-3,4-Dihydroxybuttersäure, dargestellt durch die Strukturformel (IV)

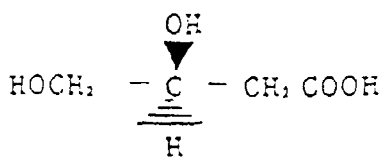

(IV)

durch Umsetzen von (R)-3-Chloro-1,2-propandiol, dargestellt durch die Strukturformel (I)

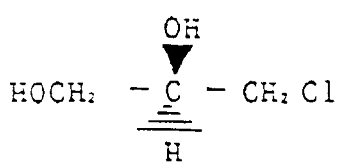

(I)

mit einem Cyanierungsmittel, wobei die Reaktion bei einer Temperatur von 40 bis 80°C in Gegenwart von 1 bis 3 molaren Äquivalenten Base und in Wasser als Lösungsmittel durchgeführt wird.

**Revendications**

1. Procédé de réaction du (R)-3-chloro-1,2-propanediol représenté par la formule développée (I)

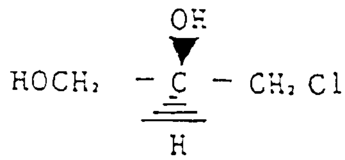

( I )

avec un agent de cyanuration, dans lequel, en contrôlant la température de réaction, une quantité d'une base selon les nécessités et une proportion d'eau dans un solvant de type alcool et/ou eau, on produit sélectivement l'un quelconque d'entre le (S)-3,4-dihydroxy-butyronitrile représenté par la formule développée (II)

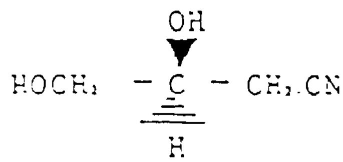

( II )

l'amide d'acide (S)-3,4-dihydroxybutyrique représenté par la formule développée (III)

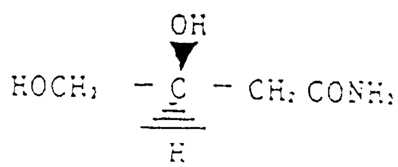

( III )

et l'acide (S)-3,4-dihydroxybutyrique représenté par la formule développée (IV)

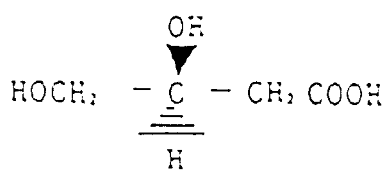

( IV )

**2.** Procédé pour la préparation sélective du (S)-3,4-dihydroxybutyronitrile réprésenté par la formule développée (II)

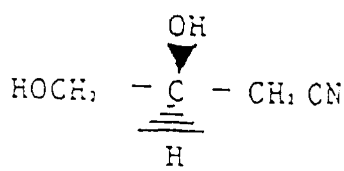

( II )

par réaction du (R)-3-chloro-1,2-propanediol représenté par la formule développée (I)

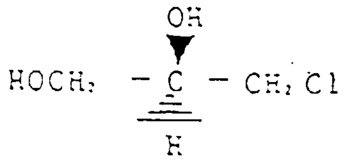

( I )

avec un agent de cyanuration, dans lequel la réaction s'effectue à une température de réaction de 20 - 40°C, en présence de 0,1 - 2 équivalents molaires de base et dans le méthanol à titre de solvant.

**3.** Procédé pour la préparation sélective de l'amide d'acide (S)-3,4-dihydroxybutyrique représenté par la formule développée (III)

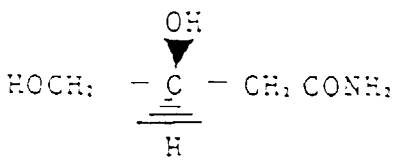

( III )

par réaction du (R)-3-chloro-1,2-propanediol représenté par la formule développée (I)

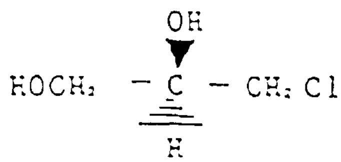

( I )

avec un agent de cyanuration, dans lequel la réaction s'effectue à une température de réaction de 30 - 60°C, dans l'eau à titre de solvant et sans l'addition d'une base.

**4.** Procédé pour la préparation sélective de l'acide (S)-3,4-dihydroxybutyrique représenté par la formule développé (IV)

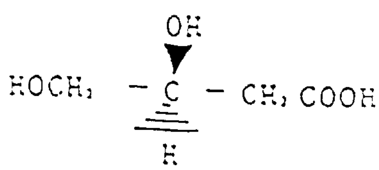

( IV )

par réaction du (R)-3-chloro-1,2-propanediol représenté par la formule développée (I)

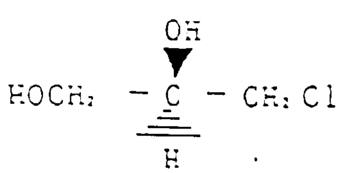

( I )

avec un agent de cyanuration, dans lequel la réaction s'effectue à une température de 40 - 80°C, en présence de 1 - 3 équivalents molaires de base et dans l'eau à titre de solvant.